# EUROPÄISCHE PATENTANMELDUNG

(11) **EP 3 482 790 A1**
(43) Veröffentlichungstag der Anmeldung: **15.05.2019**
(21) Anmeldenummer: 17201100.9
(22) Anmeldetag: 10.11.2017
(51) Int. Cl.: A61M 25/00

(54) **KATHETER ZUR INJEKTION UND/ODER APPLIKATION EINES FLUIDS**

(71) Anmelder: Bavaria Medizin Technologie GmbH, 82234 Oberpfaffenhofen (DE)
(72) Erfinder: SAUERTEIG, Knut, 86932 Pürgen/Stoffen (DE); TRUMP, Patrick, 80337 München (DE)
(74) Vertreter: Hofstetter, Schurack & Partner

(57) **Zusammenfassung**

Die Erfindung betrifft einen Katheter (10) zur Injektion und/oder Applikation eines Fluids, insbesondere eines Medikaments, mit einer Betätigungsvorrichtung (12), mit wenigstens einer Hohlnadel (60, 62, 64), welche in Längserstreckungsrichtung (X) des Katheters (10) von der Betätigungsvorrichtung (12) beabstandet ist und welche mittels der Betätigungsvorrichtung (12) bewegbar und dadurch wenigstens bereichsweise aus einer Ausführöffnung (42, 44, 46) an einem distalen Katheterende (40) des Katheters herausschiebbar ist, und mit zumindest einem Leitungselement (100), über welches die wenigstens eine Hohlnadel (60, 62, 64) mit dem Fluid versorgbar ist. Das zumindest eine Leitungselement (100) umfasst wenigstens zwei Teilbereiche (102, 104), von welchen der erste Teilbereich (102) mittels der Betätigungsvorrichtung (12) mit der wenigstens einen Hohlnadel (60, 62, 64) mitbewegbar und dabei relativ zu dem zweiten Teilbereich (104) verlagerbar ist.

## Beschreibung

Die Erfindung betrifft einen Katheter zur Injektion und/oder Applikation eines Fluids, insbesondere eines Medikaments, mit einer Betätigungsvorrichtung, mit wenigstens einer Hohlnadel, welche in Längserstreckungsrichtung des Katheters von der Betätigungsvorrichtung beabstandet ist und welche mittels der Betätigungsvorrichtung bewegbar und dadurch wenigstens bereichsweise aus einer Ausführöffnung an einem distalen Katheterende des Katheters herausschiebbar ist, und mit zumindest einem Leitungselement, über welches die wenigstens eine Hohlnadel mit dem Fluid versorgbar ist.

Aus der DE 196 33 406 A1 ist eine Betätigungsvorrichtung für einen Katheter bekannt. Die Betätigungsvorrichtung weist eine Schubhülse und einen in der Schubhülse beweglich geführten Schubstempel auf. Der Schubstempel umfasst einen Schubstempelkörper und ein Betätigungselement. Zur Arretierung des Schubstempels an der Schubhülse weist die Betätigungsvorrichtung eine Rastvorrichtung auf.

In der DE 196 34 866 A1 ist ein Katheter zur Injektion und/oder Applikation eines Fluid beschrieben, welcher einen Katheterschaft mit einem distalen Ende und einer Katheterspitze sowie mehrere Hohlnadeln umfasst. Die Hohlnadeln sind mittels einer Betätigungsvorrichtung bewegbar und an seitlichen, im Bereich des distalen Endes angeordneten Nadelaustrittsöffnungen herausschiebbar.

Die US 2007/0005018 A1 offenbart einen Katheter mit Nadeln zur direkten Injektion. Ein distales Ende des Katheters ist mittels einer, dem distalen Ende gegenüberliegenden Positioniervorrichtung durch eine Bedienperson bewegbar.

Aus der EP 2 337 605 B1 ist ein Nadelkatheter bekannt, welcher einen, innerhalb eines Katheterschaftes des Katheters angeordneten Spanndraht umfasst, mittels welchem ein distaler Schaftbereich durch Aufbringen einer Zugkraft auf den Spanndraht gebogen werden kann. Der Spanndraht ist hierzu mit dem distalen Schaftbereich verbunden.

Die US 2012/0016339 A1 beschreibt eine Vorrichtung mit einem Katheter, welcher eine Nadel zur intraoperativen, kardialen Injektion umfasst. Ein Endbereich des Katheters kann dabei mittels mehrerer, innerhalb des Katheters angeordneter Führungsdrähte in verschiedene Richtungen gebogen werden. Die Führungsdrähte sind mit jeweiligen Bewegungsmechanismen gekoppelt, welche durch eine Bedienperson manuell betätigt werden können.

Aufgabe der vorliegenden Erfindung ist es, einen Katheter der eingangs genannten Art bereitzustellen, welcher einen besonders einfachen Aufbau aufweist.

Diese Aufgabe wird durch einen Katheter mit den Merkmalen des Patentanspruchs 1 gelöst. Vorteilhafte Ausgestaltungen der Erfindung sind Gegenstand der abhängigen Ansprüche.

Die Erfindung geht von einem Katheter zur Injektion und/oder Applikation eines Fluids, insbesondere eines Medikaments, mit einer Betätigungsvorrichtung, mit wenigstens einer Hohlnadel, welche in Längserstreckungsrichtung des Katheters von der Betätigungsvorrichtung beabstandet ist und welche mittels der Betätigungsvorrichtung bewegbar und dadurch wenigstens bereichsweise aus einer Ausführöffnung an einem distalen Katheterende des Katheters herausschiebbar ist, und mit zumindest einem Leitungselement, über welches die wenigstens eine Hohlnadel mit dem Fluid versorgbar ist, aus.

Erfindungsgemäß umfasst das zumindest eine Leitungselement wenigstens zwei Teilbereiche, von welchen der erste Teilbereich mittels der Betätigungsvorrichtung mit der wenigstens einen Hohlnadel mitbewegbar und dabei relativ zu dem zweiten Teilbereich verlagerbar ist. Dies ist von Vorteil, da durch die Möglichkeit des Mitbewegens des ersten Teilbereichs mit der wenigstens einen Hohlnadel eine Verformbarkeit des Leitungselements gegeben ist, wodurch mittels dieses Leitungselements sowohl die Versorgung der wenigstens einen Hohlnadel mit dem Fluid, als auch eine Längenkompensation zum Ausgleichen eines Relativversatzes zwischen der wenigstens einen Hohlnadel und dem zweiten Teilbereich während des Bewegens der wenigstens einen Hohlnadel ermöglicht ist. Das Leitungselement erfüllt somit mehrere Funktionen, wodurch auf zusätzliche Längenausgleichselemente verzichtet werden kann und der Katheter damit insgesamt einen besonders einfachen Aufbau aufweist.

Die Betätigungsvorrichtung kann an einem Betätigungsbereich des Katheters angeordnet sein. Der Betätigungsbereich kann an einem, dem distalen Katheterende gegenüberliegenden, proximalen, körperfernen Katheterende angeordnet sein. Dadurch ist ein besonders großer Längenabschnitt des Katheters im Rahmen eines operativen Eingriffs einführbar. An dem Betätigungsbereich kann die wenigstens eine Hohlnadel mittels der Betätigungsvorrichtung betätigbar und dadurch relativ zu dem distalen, körpernahen Katheterende geführt verschiebbar sowie aus der Ausführöffnung herausschiebbar sein. Die Betätigungsvorrichtung kann dazu ausgebildet sein, die wenigstens eine Hohlnadel zumindest zwischen einer eingefahrenen Position und einer ausgefahrenen Position zu verlagern. In der eingefahrenen Position kann eine Nadelspitze der wenigstens einen Hohlnadel innerhalb des Katheters aufgenommen sein, wohingegen die Nadelspitze in der ausgefahrenen Position aus der Ausführöffnung in eine Umgebung des distalen Katheterendes ragen kann. In der ausgefahrenen Position kann dementsprechend ein Einstechen, beispielsweise in Gewebe, mittels der Nadelspitze erfolgen, wohingegen in der eingefahrenen Position beispielsweise eine unerwünschte, durch die Nadelspitze bewirkte Verletzung von Gewebe unterbunden sein kann.

An dem Betätigungsbereich kann die Betätigungsvorrichtung dementsprechend auf die wenigstens eine Hohlnadel einwirken, um diese relativ zu dem distalen Katheterende geführt zu verschieben und aus der Ausführöffnung herauszuschieben.

Bevorzugt ist die wenigstens eine Hohlnadel unter Ausübung einer Betätigungskraft auf das Leitungselement, insbesondere auf den ersten Teilbereich des Leitungselements, mittels der Betätigungsvorrichtung bewegbar. Die Betätigungskraft kann hierbei durch die Betätigungsvorrichtung auf das Leitungselement und von dem Leitungselement auf die wenigstens eine Hohlnadel übertragbar sein. Dies ermöglicht ein Bewegen der wenigstens einen Hohlnadel zusammen mit dem ersten Teilbereich sowie eine gleichzeitige Versorgung der wenigstens einen Hohlnadel mit dem Fluid über das Leitungselement unter besonders geringem baulichem Aufwand. So kann die Betätigungskraft beispielsweise als Zugkraft auf das Leitungselement ausgeübt werden, um die wenigstens eine Hohlnadel in die ausgefahrene Position zu verlagern und dabei den ersten Teilbereich von dem zweiten Teilbereich zu beabstanden. Hingegen kann die wenigstens eine Hohlnadel durch Lösen der Betätigungskraft beispielsweise von der ausgefahrenen Position in die eingefahrene Position verlagert werden.

Der erste Teilbereich kann wenigstens bereichsweise von der Betätigungsvorrichtung abragen. Dies ist von Vorteil, da die wenigstens eine Hohlnadel dementsprechend kürzer ausgebildet sein kann und sich somit lediglich über einen von der Betätigungsvorrichtung beabstandeten Teilbereich des Katheters erstrecken kann. Dies ermöglicht eine besonders einfache konstruktive Ausgestaltung des Katheters.

Der zweite Teilbereich kann zur fluidleitenden Koppelung mit einer externen Versorgungsleitung ausgebildet sein. Über die externe Versorgungsleitung kann das Fluid in das Leitungselement eingeleitet werden. Der zweite Teilbereich kann ortsfest an der Betätigungsvorrichtung fixiert sein. Durch das ortsfeste Koppeln des zweiten Teilbereichs an der Betätigungsvorrichtung kann die externe Versorgungsleitung von der Bewegung des ersten Teilbereichs entkoppelt und damit besonders aufwandsarm gelagert sein.

In einer vorteilhaften Weiterbildung der Erfindung sind die wenigstens zwei Teilbereiche zumindest im Wesentlichen parallel zueinander orientiert. Dies ist von Vorteil, da das Leitungselement hierdurch besonders wenig Bauraum einnimmt. Im Gegensatz zu gekrümmten Verbindungsschläuchen ist somit ein besonders platzsparendes Anordnen des Leitungselements ermöglicht.

Bevorzugt sind die wenigstens zwei Teilbereiche zudem axial zueinander fluchtend ausgerichtet, wodurch das Leitungselement besonders kompakt ist. Die Teilbereiche können hierbei beispielsweise in Längserstreckungsrichtung des Katheters miteinander fluchtend orientiert sein.

In einer weiteren vorteilhaften Weiterbildung der Erfindung umfasst das Leitungselement wenigstens einen Verformungsbereich, mittels welchem die wenigstens zwei Teilbereiche fluidleitend miteinander verbunden sind und welcher mittels der Betätigungsvorrichtung verformbar ist. Dies ist von Vorteil, da der Verformungsbereich ein besonders einfaches Mitbewegen des ersten Teilbereichs zusammen mit der wenigstens einen Hohlnadel ermöglicht. Der Verformungsbereich kann hierbei in Abhängigkeit von der Bewegung der wenigstens einen Hohlnadel und des ersten Teilbereichs mittels der Betätigungsvorrichtung verformbar sein. Somit können der erste Teilbereich und der zweite Teilbereich starr im Vergleich zu dem Verformungsbereich ausgebildet sein. Dadurch kann ein jeweiliges Verformen der beiden Teilbereiche und damit eine mechanische Beanspruchung der beiden Teilbereiche beim Bewegen des ersten Teilbereichs relativ zu dem zweiten Teilbereich auf ein besonders geringes Ausmaß begrenzt oder sogar vollständig unterbunden werden. Insbesondere können die beiden Teilbereiche als jeweilige, gerade Rohrstücke ausgebildet sein.

Bevorzugt ist der Verformungsbereich elastisch verformbar. Hierdurch kann ein aufwandsarmes Entspannen des Verformungsbereichs in dessen Ausgangsform erfolgen, sobald ein Einwirken der Betätigungsvorrichtung auf den Verformungsbereich beendet wird.

In einer weiteren vorteilhaften Weiterbildung der Erfindung ist der Verformungsbereich zumindest bereichsweise helixförmig ausgebildet. Dies ist von Vorteil, da durch das helixförmige Ausbilden eine Verformung des Verformungsbereichs in verschiedene Raumrichtungen, also beispielsweise in Längserstreckungsrichtung und/oder Radialerstreckungsrichtung des Katheters, unter besonders geringem Kraftaufwand ermöglicht ist. Von besonderem Vorteil ist, dass durch das zumindest bereichsweise helixförmige Ausbilden des Verformungsbereichs ein besonders ruckfreies Mitbewegen des ersten Teilbereichs mit der wenigstens einen Hohlnadel und relativ zu dem zweiten Teilbereich ermöglicht ist.

Der Verformungsbereich kann also zumindest bereichsweise eine Helixkontur aufweisen, deren Mittelachse beispielsweise in Längserstreckungsrichtung des Katheters orientiert sein kann. Durch die Betätigungsvorrichtung kann die Helixkontur dann zumindest bereichsweise in Längserstreckungsrichtung gestreckt werden (Verformen des Verformungsbereichs), um den ersten Teilbereich relativ zu dem zweiten Teilbereich zu verlagern, also die beiden Teilbereiche voneinander zu beabstanden.

In einer weiteren vorteilhaften Weiterbildung der Erfindung umfasst die Betätigungsvorrichtung ein drehbares Betätigungselement, welches zumindest mittelbar mit dem ersten Teilbereich gekoppelt ist und durch dessen Drehung die wenigstens eine Hohlnadel mit dem ersten Teilbereich mitbewegbar ist. Dies ist von Vorteil, da durch die Drehung des Betätigungselements ein besonders ruckfreies Bewegen des ersten Teilbereichs und damit der wenigstens einen Hohlnadel ermöglicht ist. Das Betätigungselement kann beispielsweise als Handrad ausgebildet sein, wodurch ein besonders aufwandsarmes Bewegen der wenigstens einen Hohlnadel ermöglicht ist.

In einer weiteren vorteilhaften Weiterbildung der Erfindung ist das Betätigungselement relativ zu dem ersten Teilbereich drehbar mit diesem gekoppelt. Dies ist von Vorteil, da hierdurch beim Drehen des Betätigungselements eine Torsionsbeanspruchung des Leitungselements zumindest weitgehend unterbunden werden kann. Hierzu kann das Betätigungselement beispielsweise mittels eines Verbindungsstücks, welches drehfest mit dem Betätigungselement und drehbar mit dem ersten Teilbereich gekoppelt sein kann, mit dem ersten Teilbereich verbunden sein. Somit kann eine Drehung des Betätigungselements in eine translatorische Bewegung des ersten Teilbereichs umgesetzt werden, ohne das Leitungselement dabei zu tordieren.

In einer weiteren vorteilhaften Weiterbildung der Erfindung umfasst die Betätigungsvorrichtung ein Gewinde, mit welchem das Betätigungselement bei dessen Drehung in Eingriff ist und dadurch zusammen mit dem ersten Teilbereich und der wenigstens einen Hohlnadel translatorisch mitbewegbar ist. Dies ist von Vorteil, da das Gewinde ein besonders ruckfreies Bewegen des ersten Teilbereichs und der wenigstens einen Hohlnadel ermöglicht.

In einer weiteren vorteilhaften Weiterbildung der Erfindung umfasst die Betätigungsvorrichtung wenigstens ein Verriegelungselement, mittels welchem die Drehung des Betätigungselements blockierbar ist. Dies ist von Vorteil, da mittels des Verriegelungselements ein Festlegen des Betätigungselements und dadurch ein Unterbinden einer Relativbewegung zwischen der wenigstens einen Hohlnadel und dem distalen Katheterende ermöglicht ist. Dies gestattet einen besonders sicheren Betrieb des Katheters, sodass beispielsweise eine unbeabsichtigte, durch die wenigstens eine Hohlnadel bewirkte Gewebeperforation vermieden werden kann.

In einer weiteren vorteilhaften Weiterbildung der Erfindung ist der erste Teilbereich formschlüssig und/oder stoffschlüssig und/oder kraftschlüssig mit der wenigstens einen Hohlnadel verbunden. Ein stoffschlüssiges Verbinden kann beispielsweise durch Verkleben, Verschweißen und/oder Verlöten der zumindest einen Hohlnadel mit dem ersten Teilbereich bewirkt werden. Ein formschlüssiges Verbinden kann beispielsweise dadurch erzielt werden, dass der erste Teilbereich zumindest bereichsweise eine mit der zumindest einen Hohlnadel korrespondierende Anlagefläche aufweist, an welcher die Hohlnadel beispielsweise eingehakt und/oder hinterhakt sein kann. Ein kraftschlüssiges Verbinden kann beispielsweise durch Pressfügen der wenigstens einen Hohlnadel mit dem ersten Teilbereich bewirkt werden.

In einer weiteren vorteilhaften Weiterbildung der Erfindung umfasst der Katheter wenigstens einen Spülanschluss, welcher in Quererstreckungsrichtung des Leitungselements in dieses mündet. Dies ist von Vorteil, da über den Spülanschluss eine seitliche Fluideinleitung in das Leitungselement ermöglicht ist. Der Spülanschluss kann hierzu in eine seitliche Öffnung an einer Leitungswand des Leitungselements münden, wodurch insgesamt und in vorteilhafter Weise an verschiedenen Stellen eine Fluidzuführung ermöglicht ist.

Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen.

Im Folgenden sind Ausführungsbeispiele der Erfindung beschrieben. Hierzu zeigt:
- Fig. 1: eine Perspektivansicht eines Katheters;
- Fig. 2: eine vergrößerte Darstellung eines in Fig. 1 umrandeten Bereichs A, wobei ein 1-lumiges Rohr des Katheters gezeigt ist, welches ein als 4-lumiges Rohr ausgebildetes Führungselement des Katheters sowie in dem Führungselement gelagerte Hohlnadeln des Katheters umgibt;
- Fig. 3: eine weitere vergrößerte Darstellung des Bereichs A, welche einen inneren Aufbau des Katheters ohne das 1-lumige Rohr zeigt;
- Fig. 4: eine vergrößerte Darstellung des Führungselements, an welchem ein vorliegend als Draht ausgebildetes Sicherungselement eingehakt ist;
- Fig. 5: eine vergrößerte Darstellung eines distalen Katheterendes des Katheters, an welchem das Sicherungselement befestigt ist;
- Fig. 6: eine schematische Seitenansicht auf einen Teilbereich des Katheters, wobei das distale Katheterende sowie das von dem distalen Katheterende beabstandete Führungselement des Katheters gezeigt ist;
- Fig. 7: eine vergrößerte Seitenansicht des distalen Katheterendes, wobei die Befestigung des Sicherungselements an dem distalen Katheterende gezeigt ist;
- Fig. 8: eine Draufsicht auf eine Katheterspitze des distalen Katheterendes gemäß einer in Fig. 7 angegebenen Ansichtsrichtung B
- Fig. 9: eine perspektivische Schnittdarstellung einer Betätigungsvorrichtung des Katheters;
- Fig. 10: eine Perspektivansicht der Betätigungsvorrichtung, wobei ein Verriegelungselement eine Drehung eines Betätigungselements der Betätigungsvorrichtung blockiert;
- Fig. 11: eine weitere Perspektivansicht der Betätigungsvorrichtung, wobei das Verriegelungselement die Drehung des Betätigungselements der Betätigungsvorrichtung freigibt; und
- Fig. 12: eine weitere Perspektivansicht der Betätigungsvorrichtung.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche und funktionsgleiche Elemente jeweils mit denselben Bezugszeichen versehen.

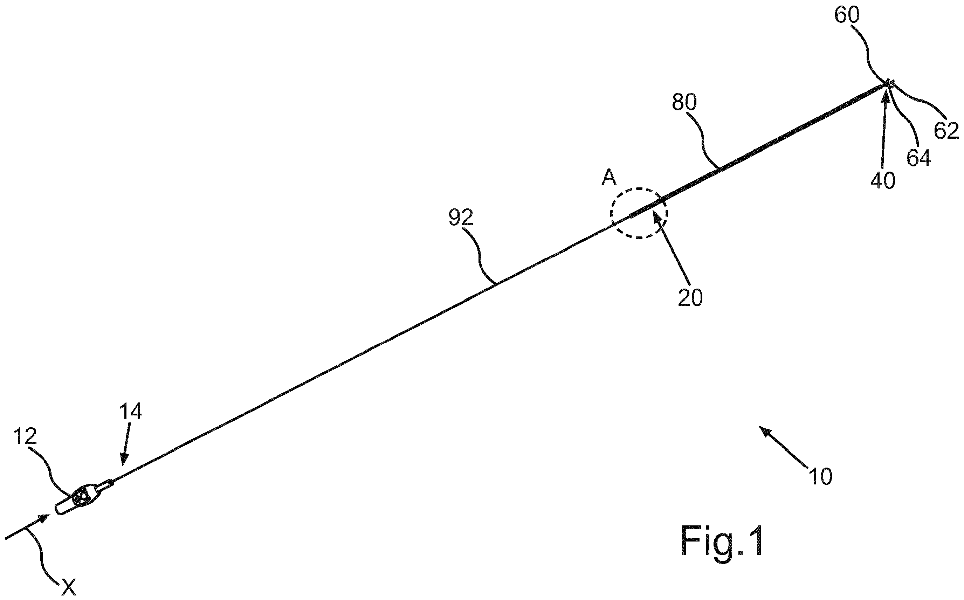

Fig. 1 zeigt eine Perspektivansicht eines Katheters 10, welcher zur Injektion und/oder Applikation eines Fluids, insbesondere eines Medikaments ausgestaltet ist. Der Katheter 10 ist vorliegend vollständig aus biokompatiblen Werkstoffen gebildet, zu welchen biokompatible Metalle, biokompatible Kunststoffe und biokompatible Keramiken gehören können, um nur einige Beispiele zu nennen.

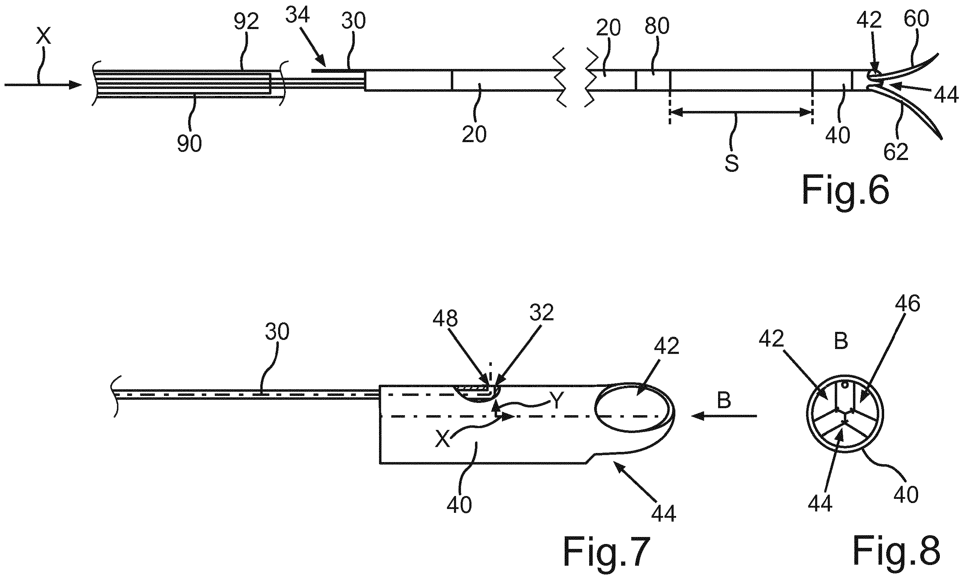

Der Katheter 10 umfasst mehrere Hohlnadeln 60, 62, 64, welche an einem körpernahen, distalen Katheterende 40 des Katheters 10 über in Fig. 8 vergrößert dargestellte Ausführöffnungen 42, 44, 46 aus einem Katheterinnenraum des Katheters 10 herausschiebbar sind.

Der Katheter 10 umfasst zudem eine Betätigungsvorrichtung 12, welche an einem, dem distalen Katheterende 40 gegenüberliegenden, körperfernen Ende (proximalen Katheterende) angeordneten Betätigungsbereich 14 des Katheters 10 angeordnet ist. Die Betätigungsvorrichtung 12 ist in Längserstreckungsrichtung X des Katheters 10 von den Hohlnadeln 60, 62, 64 beabstandet. Die Hohlnadeln 60, 62, 64 sind über ein Leitungselement 100 mit dem Fluid versorgbar. Das Leitungselement 100 erstreckt sich dabei zumindest bereichsweise durch ein Vorrichtungsgehäuse 13 der Betätigungsvorrichtung 12. Die Längserstreckungsrichtung X ist beispielsweise in Fig. 1, Fig. 6, Fig. 7 und Fig. 9 gezeigt. Die Längserstreckungsrichtung X ist vorliegend durch einen Pfeil verdeutlicht, welcher auch eine Einführrichtung des Katheters 10 angibt, in welcher der Katheter 10 in eine Körperöffnung, wie beispielsweise eine Arterie, einführbar ist.

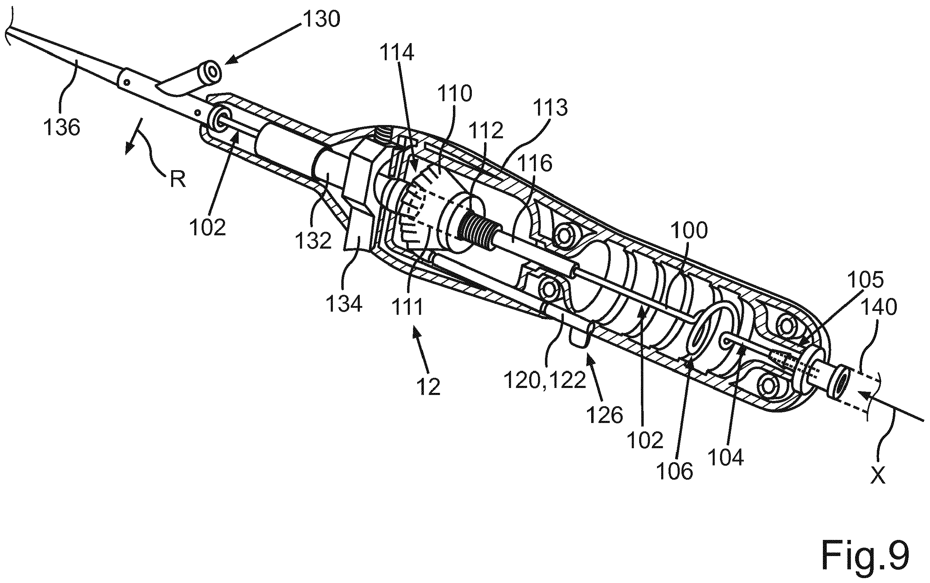

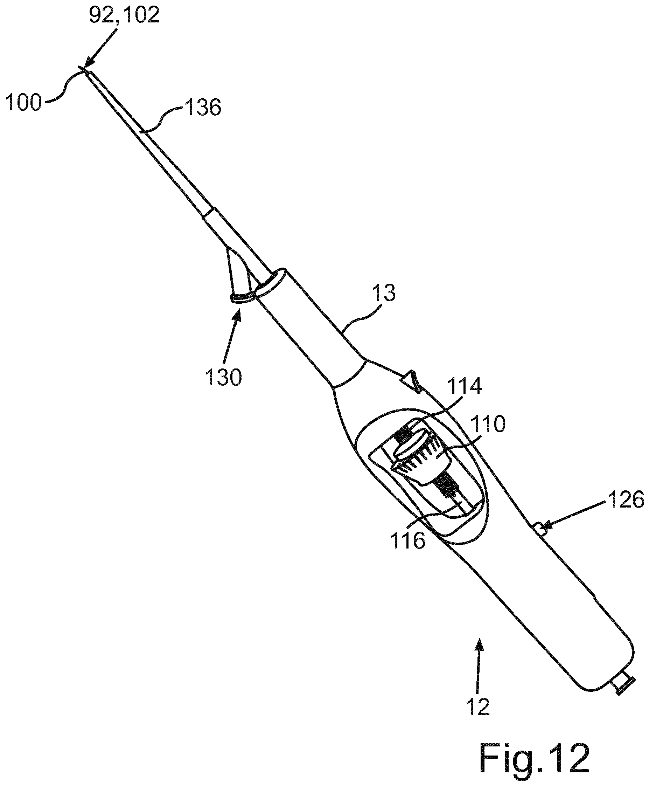

Das Leitungselement 100 umfasst zwei Teilbereiche 102, 104, von welchen der erste Teilbereich 102 mittels der Betätigungsvorrichtung 12 mit den Hohlnadeln 60, 62, 64 mitbewegbar und dabei relativ zu dem zweiten Teilbereich 104 verlagerbar ist. Die beiden Teilbereiche 102, 104 sind vorliegend parallel zueinander orientiert und fluchten zudem in Längserstreckungsrichtung X des Katheters miteinander. Der Katheter 10 umfasst einen in Fig. 9 und Fig. 12 erkennbaren Spülanschluss 130, welcher an dem Vorrichtungsgehäuse 13 befestigt ist und in Quererstreckungsrichtung R des Leitungselements 100 in dieses mündet. Über den Spülanschluss 130 kann eine seitliche Fluidzuführung in das Leitungselement 100 erfolgen, wodurch ein Spülen des Leitungselements 100 ermöglicht ist.

Zum Bewegen des ersten Teilbereichs 102 umfasst die Betätigungsvorrichtung 12 ein drehbares Betätigungselement 110, welches vorliegend als Handrad ausgestaltet ist. Der erste Teilbereich 102 ist durch eine, in Fig. 9 gestrichelt angedeutete Betätigungselementöffnung 111 des Betätigungselements 110 hindurchgeführt. Das Betätigungselement 110 ist vorliegend über ein Verbindungsstück 116 relativ zu dem ersten Teilbereich 102 drehbar mit diesem gekoppelt. Das Verbindungsstück 116 ist dabei drehfest mit dem Betätigungselement 110 und drehbar mit dem ersten Teilbereich 102 gekoppelt. Das Verbindungsstück 116 ermöglicht dadurch eine Kraftübertragung zwischen dem Betätigungselement 110 und dem ersten Teilbereich 102, wohingegen eine Übertragung von Torsionsmomenten zwischen dem Betätigungselement 110 und dem ersten Teilbereich 102 unterbunden ist.

Das Verbindungsstück 116 ist vorliegend als 1-lumige Hülse ausgestaltet, durch welche sich der erste Teilbereich 102 erstreckt, und welche an einem, an der Betätigungselementöffnung 111 vorgesehenen Innengewinde 112 mit dem Betätigungselement 110 verschraubt ist.

Über das Innengewinde 112 steht das Betätigungselement 110 zudem in permanentem Eingriff mit einem Gewinde 114 der Betätigungsvorrichtung 12. Das Gewinde 114 ist dementsprechend als Außengewinde ausgebildet.

Durch Drehen des Betätigungselements 110 kann dieses auf das Gewinde 114 aufgeschraubt, bzw. von dem Gewinde 114 heruntergeschraubt werden.

Beim Aufschrauben des Betätigungselements 110 auf das Gewinde 114 kann über das Verbindungsstück 116 eine Betätigungskraft in Form einer Zugkraft auf den ersten Teilbereich 102 ausgeübt werden um den ersten Teilbereich 102 relativ zu dem zweiten Teilbereich 104 zu verlagern und die beiden Teilbereiche 102, 104 dementsprechend voneinander zu beabstanden.

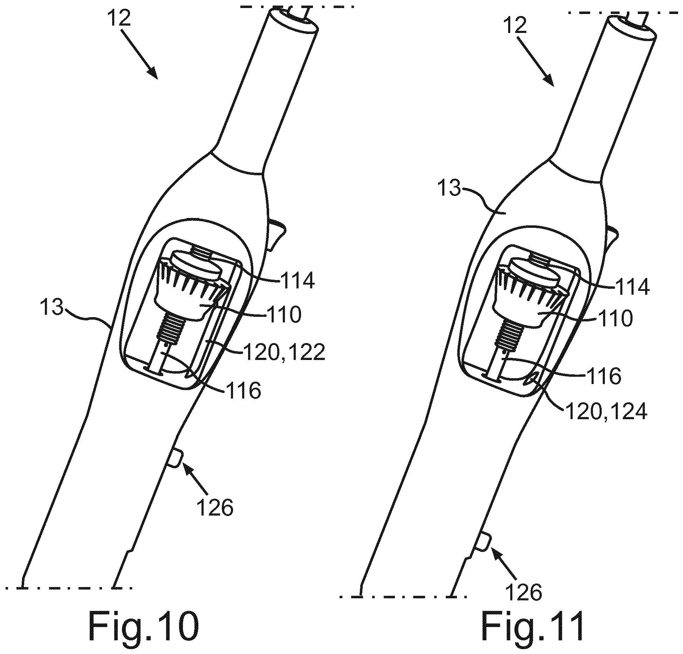

Durch das Aufschrauben kann das Betätigungselement 110 also relativ zu dem in den Fig. 10, Fig. 11 und Fig. 12 erkennbaren Gewinde 114 gedreht werden um die Hohlnadeln 60, 62, 64 zusammen mit dem ersten Teilbereich 102 zu bewegen, ohne dass es dabei zu einer unzulässigen Torsion des Leitungselements 100 kommt. Beim Drehen des Betätigungselements 110 werden die Hohlnadeln 60, 62, 64 zusammen mit dem ersten Teilbereich 102 zusammen mit dem ersten Teilbereich 102 translatorisch entlang der Längserstreckungsrichtung X verschoben und dabei relativ zu dem zweiten Teilbereich 104 bewegt.

Das Gewinde 114 (Außengewinde) ist an einem Leitungsführungselement 132 angeordnet. Das Leitungsführungselement 132 umfasst eine Leitungsdurchgangsöffnung, durch welche zumindest der erste Teilbereich 102 des Leitungselements 100 hindurchgeführt ist. Der erste Teilbereich 102 erstreckt sich somit insgesamt durch das Verbindungsstück 116, das Betätigungselement 110, und das Leitungsführungselement 132, wobei der erste Teilbereich 102 in Längserstreckungsrichtung X aus dem Vorrichtungsgehäuse 13 in Richtung des distalen Katheterendes 40 herausragt. Das Leitungsführungselement 132 ist über ein federbelastetes Klemmelement 134 gegenüber dem Vorrichtungsgehäuse 13 verspannt, wie ebenfalls in Fig. 9 gezeigt ist.

Um eine unerwünschte Drehung des Betätigungselements 110 zu blockieren, umfasst die Betätigungsvorrichtung 12 ein an dem Vorrichtungsgehäuse 13 verschiebbar gelagertes Verriegelungselement 120, welches vorliegend stiftförmig ausgebildet ist. Das Verriegelungselement 120 umfasst einen Handgriffbereich 126, an welchem das Verriegelungselement 120 vorliegend in Längserstreckungsrichtung X zwischen einer in Fig. 10 gezeigten Verriegelungsstellung 122 und einer in Fig. 11 gezeigten Entriegelungsstellung 124 verschiebbar ist. In der Verriegelungsstellung 122 ist das Verriegelungselement 120 mit dem Betätigungselement 110 in Eingriff und blockiert dadurch die Drehung des Betätigungselements 110 relativ zu dem Vorrichtungsgehäuse 13. In der Entriegelungsstellung 124 ist die Drehung des Betätigungselements 110 hingegen freigegeben.

Fig. 9 zeigt, dass an dem zweiten Teilbereich 104 eine vorliegend gestrichelt angedeutete, externe Versorgungsleitung 140 angeschlossen werden kann, über welche das Fluid in das Leitungselement 100 einleitbar und dadurch den Hohlnadeln 60, 62, 64 zuführbar ist.

Um das relative Bewegen des ersten Teilbereichs 102 zu dem zweiten Teilbereich 104 zu erleichtern, umfasst das Leitungselement 100 einen helixförmig ausgebildeten Verformungsbereich 106, mittels welchem die wenigstens zwei Teilbereiche 102, 104 fluidleitend miteinander verbunden sind und welcher durch Drehen des Betätigungselements 110 auf dem Gewinde 114 verformbar ist. Der helixförmig ausgebildete Verformungsbereich 106 kann durch Ausüben der Betätigungskraft auf den ersten Teilbereich 102 zumindest bereichsweise auseinandergezogen werden, sodass der erste Teilbereich 102 in Längserstreckungsrichtung X relativ zu dem zweiten Teilbereich 104 verlagert und dadurch von dem zweiten Teilbereich 104 beabstandet wird.

Der erste Teilbereich 102 ist vorliegend zumindest bereichsweise als Katheterschaft 90 ausgebildet, in welchem die Hohlnadeln 60, 62, 64 aufgenommen und festgelegt sind.

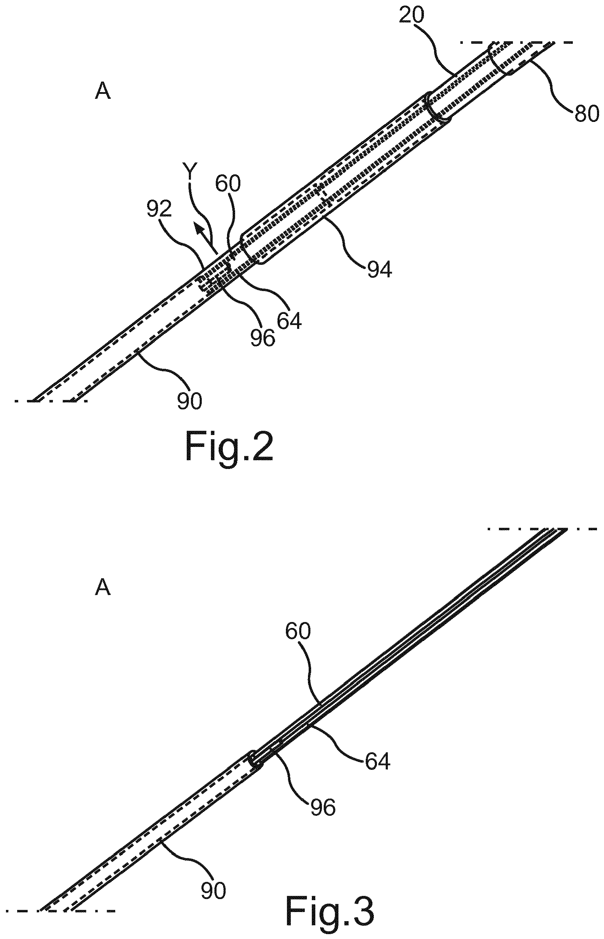

Der Katheterschaft 90 ist beispielsweise in Fig. 2 und Fig. 3 vergrößert dargestellt. Im vorliegenden Ausführungsbeispiel sind die Hohlnadeln 60, 62, 64 stoffschlüssig und kraftschlüssig mit dem ersten Teilbereich 102 verbunden. Zusätzlich oder alternativ ist auch eine formschlüssige Verbindung des ersten Teilbereichs 102 mit den jeweiligen Hohlnadeln 60, 62, 64 denkbar.

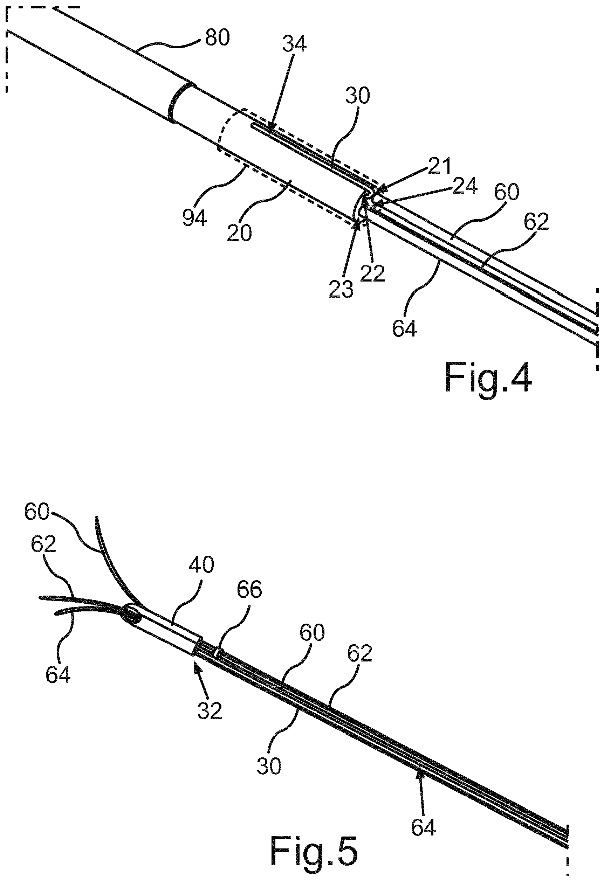

Über den Katheterschaft 90 und das Verbindungsstück 116 ist das Betätigungselement 110 somit vorliegend mechanisch mit den Hohlnadeln 60, 62, 64 gekoppelt, sodass die Hohlnadeln 60, 62, 64 durch Drehen des Betätigungselements 110, zwischen einer jeweiligen, eingefahrenen Position und einer jeweiligen, ausgefahrenen Position verlagert werden können. Die Hohlnadeln 60, 62, 64 sind jeweils vorgebogen, sodass die Hohlnadeln 60, 62, 64 in der ausgefahrenen Position voneinander abgespreizt sind, wie beispielsweise aus Fig. 5 hervorgeht. Die Hohlnadeln 60, 62, 64 sind durch einen Abstandshalter 66 hindurchgeführt und dadurch auch bei deren Bewegung in einem konstanten Abstand zueinander gehalten.

Aus der Zusammenschau von Fig. 1 mit Fig. 2 oder Fig. 4 ist erkennbar, dass der Katheter 10 ein Führungselement 20 umfasst. Das Führungselement 20 ist vorliegend als zylindrisches Rohr ausgebildet und umfasst insgesamt vier Lumen, nämlich ein erstes Lumen 21, ein zweites Lumen 22, ein drittes Lumen 23 und ein viertes Lumen 24 (siehe Fig. 4). Mit anderen Worten ist das Führungselement 20 vorliegend als 4-lumiges Rohr ausgestaltet. Die Lumen 21, 22, 23, 24 sind dabei regelmäßig über das Führungselement 20 verteilt und erstrecken sich vorliegend in Längserstreckungsrichtung X des Katheters 10.

Beim Einführen des Katheters 10 wirkt eine Druckkraft entgegen der Pfeilrichtung auf das distale Katheterende 40, wohingegen beim Herausziehen des Katheters 10 eine, in Pfeilrichtung orientierte Zugkraft auf das distale Katheterende 40 wirkt.

Durch Betätigung der Betätigungsvorrichtung 12 sind die Hohlnadeln 60, 62, 64, welche vorliegend in den Lumen 21, 23, 24 aufgenommen sind, relativ zu dem Führungselement 20 geführt in Längserstreckungsrichtung X verschiebbar und wenigstens bereichsweise aus den jeweiligen Ausführöffnungen 42, 44, 46 im Bereich des distalen Katheterendes 40 herausschiebbar.

Fig. 2 zeigt zudem einen beispielhaften Aufbau des Katheters 10 aus mehreren verschiedenen Komponenten. Das Führungselement 20 ist dabei bereichsweise durch ein 1-lumiges Rohr 94 umgeben. Das 1-lumige Rohr 94 umgibt des Weiteren den Katheterschaft 90, in welchem die Hohlnadeln 60, 62, 64 des Katheters 10 geführt und befestigt sind, bereichsweise. Der Katheterschaft 90 kann auch als "hypotube" bezeichnet werden.

Der Katheterschaft 90 und der erste Teilbereich 102 sind zudem durch ein Flechtrohr 92, welches auch als "braided tubing" bezeichnet werden kann, umgeben. Das Flechtrohr 92 umfasst ein Geflecht und eine Kunststoffummantelung, in welche das Geflecht eingebettet ist. Das Flechtrohr 92 ist bereichsweise zwischen dem Katheterschaft 90 und dem 1-lumigen Rohr 94 angeordnet und endet in Längserstreckungsrichtung X einerseits an dem Führungselement 20 und andererseits an der Betätigungsvorrichtung 12, wie aus der Zusammenschau von Fig. 1 und Fig. 2 hervorgeht. Das Flechtrohr 92 ist durch eine in Fig. 9 und Fig. 12 gezeigt Führungshülse 136 hindurchgeführt am Vorrichtungsgehäuse 13 befestigt. Die Führungshülse 136 ist an dem Spülanschluss 130 befestigt.

Durch Betätigung der Betätigungsvorrichtung 12 ist der Katheterschaft 90 zusammen mit den Hohlnadeln 60, 62, 64 innerhalb des Flechtrohres 92 und innerhalb des 1-lumigen Rohres 94 und damit relativ zu dem 1-lumigen Rohr 94 verschiebbar. Das Führungselement 20 begrenzt dabei die Verschiebbarkeit in Längserstreckungsrichtung X. Die Hohlnadeln 60, 62, 64 können also zusammen mit dem Katheterschaft 90 (und damit mit dem ersten Teilbereich 102) in der Längserstreckungsrichtung X verschoben werden, bis der Katheterschaft 90 an dem Führungselement 20 anstößt und sich die Hohlnadeln 60, 62, 64 dann dementsprechend in der ausgefahrenen Position befinden. Der Katheterschaft 90 kann - ebenso wie die Hohlnadeln 60, 62, 64 - beispielsweise aus einem Formgedächtnismaterial, beispielsweise aus NiTi-NOL, gebildet sein.

Um einen möglichst ausfallsicheren Betrieb des Katheters 10 sicherzustellen, umfasst dieser wenigstens ein als Draht, als Seil oder als Band ausgebildetes Sicherungselement 30, mittels welchem das distale Katheterende 40 an dem zumindest einen Führungselement 20 verliersicher festgelegt ist. Vorliegend ist das Sicherungselement 30 als Draht ausgebildet, wie in Fig. 4 bis Fig. 7 erkennbar ist. Das Sicherungselement 30 ist vorliegend in einem jeweiligen Innenraum des distalen Katheterendes 40 sowie des Führungselements 20 und somit im Katheterinnenraum des Katheters 10 angeordnet.

Das Sicherungselement 30 kann stoffschlüssig und zusätzlich formschlüssig mit dem distalen Katheterende 40 verbunden sein, wie durch einen in Fig. 7 dargestellten Ausbruch an einem Wandbereich des distalen Katheterendes 40 erkennbar ist. Das distale Katheterende 40 umfasst an diesem Wandbereich eine Aufnahmeöffnung 48, in welche ein erstes Sicherungselementende 32 des wenigstens einen Sicherungselements 30 eingeführt und mit dem distalen Katheterende 40 verbunden ist. Das Sicherungselementende 32 ist dabei durch Verhaken formschlüssig an der Aufnahmeöffnung 48 mit dem distalen Katheterende 40 verbunden. Die Aufnahmeöffnung 48 erstreckt sich vorliegend in einer, zur Längserstreckungsrichtung X senkrechten Radialerstreckungsrichtung Y des Katheters 10. Die Radialerstreckungsrichtung Y entspricht vorliegend der Quererstreckungsrichtung R des Leitungselements 100.

Die Aufnahmeöffnung 48 kann durch einen Kragen (nicht dargestellt) begrenzt sein, wodurch das Sicherungselement 30 besonders verliersicher an dessen erstem Sicherungselementende 32 in der Aufnahmeöffnung 48 festgelegt sein kann.

Das Sicherungselement 30 ist des Weiteren an einem, dem ersten Sicherungselementende 32 gegenüberliegenden, zweiten Sicherungselementende 34 des Sicherungselements 30 mit dem Führungselement 20 verhakt. Die Zusammenschau von Fig. 4 und Fig. 6 zeigt, dass das Sicherungselement 30 mit dem zweiten Sicherungselementende 34 durch das zweite Lumen 22 des Führungselements 20 hindurchgeführt und das zweite Sicherungselementende 34 nach dem Durchführen umgebogen und dadurch mit dem Führungselement 20 verhakt ist. Fig. 6 zeigt das zweite Sicherungselementende 34 im unverformten, also noch nicht umgebogenen Zustand, wohingegen in Fig. 4 das umgebogene, zweite Sicherungselementende 34 dargestellt ist. Das 1-lumige Rohr 94 umgibt das umgebogene Sicherungselementende 34 ebenso wie das Führungselement 20. Um dies zu verdeutlichen ist das 1-lumige Rohr 94 nochmals gestrichelt in Fig. 4 angedeutet.

Fig. 6 zeigt, dass das Führungselement 20 in Längserstreckungsrichtung X des Katheters 10 in einem Abstand s zu dem distalen Katheterende 40 angeordnet ist. Der lediglich in Fig. 5 dargestellte Abstandshalter 66 kann beim Bewegen der Hohlnadeln 60, 62, 64 zumindest bereichsweise über den Abstand s bewegt werden, wenngleich dies nicht aus Fig. 6 hervorgeht.

Sowohl das Führungselement 20, als auch das distale Katheterende 40 sind zumindest bereichsweise durch ein vorliegend transparentes, als Kunststoffschlauch ausgebildetes Hüllelement 80 des Katheters 10 umschlossen. Das Hüllelement 80 ist dabei einerseits mit dem Führungselement 20 und andererseits mit dem distalen Katheterende 40 verbunden.

Bevorzugt kann das zumindest eine Hüllelement 80 zumindest das Sicherungselement 30 wenigstens über den Abstand s zwischen dem Führungselement 20 und dem distalen Katheterende 40 hinweg zumindest bereichsweise kontaktfrei umgeben. Dadurch kann ein besonders reibungsarmes elastisches Verformen des Katheters 10 bei dessen Einführen in zu behandelndes Gewebe erfolgen. Ein Reibkontakt zwischen dem Sicherungselement 30 und dem Hüllelement 80 kann somit zumindest bereichsweise vermieden werden.

Das Hüllelement 80 kann auch die Hohlnadeln 60, 62, 64 zumindest über den Abstand s hinweg kontaktfrei über deren Nadelumfang umgeben, sodass ein besonders reibungsarmes Bewegen der Hohlnadeln 60, 62, 64 zwischen der eingefahrenen Position und der ausgefahrenen Position erfolgen kann.

Fig. 3 zeigt, dass der Katheter 10 unter besonders geringem Aufwand umgerüstet werden kann, so dass beispielsweise lediglich zwei Hohlnadeln 60, 64 statt der zuvor beschriebenen drei Hohlnadeln 60, 62, 64 vorgesehen sein können. Zu diesem Zweck können nicht mit Nadeln versehe Lumen mit einem in Fig. 3 angedeuteten Füllstift 96 verfüllt werden, um eine Menge an benötigtem Klebstoff zu verringern, also ein mit Klebstoff aufzufüllendes Klebevolumen zu reduzieren. Dadurch kann ein unerwünschter Fluidrückfluss in Richtung der Betätigungsvorrichtung 12 bei der Verwendung des Katheters 10 unterbunden werden.

## Patentansprüche

1. Katheter (10) zur Injektion und/oder Applikation eines Fluids, insbesondere eines Medikaments, mit einer Betätigungsvorrichtung (12), mit wenigstens einer Hohlnadel (60, 62, 64), welche in Längserstreckungsrichtung (X) des Katheters (10) von der Betätigungsvorrichtung (12) beabstandet ist und welche mittels der Betätigungsvorrichtung (12) bewegbar und dadurch wenigstens bereichsweise aus einer Ausführöffnung (42, 44, 46) an einem distalen Katheterende (40) des Katheters herausschiebbar ist, und mit zumindest einem Leitungselement (100), über welches die wenigstens eine Hohlnadel (60, 62, 64) mit dem Fluid versorgbar ist,
**dadurch gekennzeichnet, dass**
das zumindest eine Leitungselement (100) wenigstens zwei Teilbereiche (102, 104) umfasst, von welchen der erste Teilbereich (102) mittels der Betätigungsvorrichtung (12) mit der wenigstens einen Hohlnadel (60, 62, 64) mitbewegbar und dabei relativ zu dem zweiten Teilbereich (104) verlagerbar ist.

2. Katheter (10) nach Anspruch 1,
**dadurch gekennzeichnet, dass**
die wenigstens zwei Teilbereiche (102, 104) zumindest im Wesentlichen parallel zueinander orientiert sind.

3. Katheter (10) nach Anspruch 1 oder 2,
**dadurch gekennzeichnet, dass**
das Leitungselement (100) wenigstens einen Verformungsbereich (106) umfasst, mittels welchem die wenigstens zwei Teilbereiche (102, 104) fluidleitend miteinander verbunden sind und welcher mittels der Betätigungsvorrichtung (12) verformbar ist.

4. Katheter (10) nach Anspruch 3,
**dadurch gekennzeichnet, dass**
der Verformungsbereich (106) zumindest bereichsweise helixförmig ausgebildet ist.

5. Katheter (10) nach einem der vorhergehenden Ansprüche,
**dadurch gekennzeichnet, dass**
die Betätigungsvorrichtung (12) ein drehbares Betätigungselement (110) umfasst, welches zumindest mittelbar mit dem ersten Teilbereich (102) gekoppelt ist und durch dessen Drehung die wenigstens eine Hohlnadel (60, 62, 64) mit dem ersten Teilbereich (102) mitbewegbar ist.

6. Katheter (10) nach Anspruch 5,
**dadurch gekennzeichnet, dass**
das Betätigungselement (110) relativ zu dem ersten Teilbereich (102) drehbar mit diesem gekoppelt ist.

7. Katheter (10) nach einem der Ansprüche 5 oder 6,
**dadurch gekennzeichnet, dass**
die Betätigungsvorrichtung (12) ein Gewinde (114) umfasst, mit welchem das Betätigungselement (110) bei dessen Drehung in Eingriff ist und dadurch zusammen mit dem ersten Teilbereich (104) und der wenigstens einen Hohlnadel (60, 62, 64) translatorisch mitbewegbar ist.

8. Katheter (10) nach einem der Ansprüche 5 bis 7,
**dadurch gekennzeichnet, dass**
die Betätigungsvorrichtung (12) wenigstens ein Verriegelungselement (120) umfasst, mittels welchem die Drehung des Betätigungselements (110) blockierbar ist.

9. Katheter (10) nach einem der vorhergehenden Ansprüche,
**dadurch gekennzeichnet, dass**
der erste Teilbereich (102) formschlüssig und/oder stoffschlüssig und/oder kraftschlüssig mit der wenigstens einen Hohlnadel (60, 62, 64) verbunden ist.

10. Katheter (10) nach einem der vorhergehenden Ansprüche,
**dadurch gekennzeichnet, dass**
der Katheter (10) wenigstens einen Spülanschluss (130) umfasst, welcher in Quererstreckungsrichtung (R) des Leitungselements (100) in dieses mündet.
